# EUROPEAN PATENT SPECIFICATION

(11) **EP 3 784 313 B1**
(45) Date of publication and mention of the grant of the patent: **27.08.2025**
(21) Application number: 19791452.6
(22) Date of filing: 19.04.2019
(51) Int. Cl.: A61M 5/31, A61M 5/142, A61M 5/32, A61M 5/34, A61M 5/165, B01D 15/00

(54) **DELIVERY DEVICE AND ADSORBENT**
ABGABEVORRICHTUNG UND ADSORPTIONSMITTEL
DISPOSITIF D'ADMINISTRATION ET ADSORBANT

(30) Priority: 27.04.2018 US 201862663605 P
(43) Date of publication of application: 03.03.2021
(73) Proprietor: Becton, Dickinson and Company, Franklin Lakes, NJ 07417-1880 (US)
(72) Inventor: NOVAK, Matthew, Durham, North Carolina 27701 (US); MAINZ, Emilie, Malden, Massachusetts 02148 (US); RINI, Christopher, Raleigh, North Carolina 27617 (US); ROBERTS, Bruce, Franklin Lakes, New Jersey 07417 (US)
(74) Representative: dompatent von Kreisler Selting Werner - Partnerschaft von Patent- und Rechtsanwälten mbB
(86) International application number: PCT/US2019/028248
(87) International publication number: WO 2019/209644

(56) References cited:
- US-A- 5 531 683
- US-A1- 2002 060 180
- US-A1- 2011 203 585
- US-A1- 2012 253 022
- US-A1- 2015 283 032
- US-A1- 2016 354 542
- US-A1- 2016 354 542
- US-A1- 2017 232 204

## Description

This application claims priority to U.S. Provisional Patent Application No. 62/663,605, filed on April 27, 2018.

### Field of the Invention

The present invention relates generally to a delivery device for delivering a substance to a patient and filtering or removing selected compounds from the substance before delivery to the patient. The delivery device includes an adsorbent material positioned upstream of a delivery member, such as a cannula or catheter, to remove selected compounds from the substance just prior injecting into the patient. The delivery device in one embodiment is suitable for delivering a controlled dosage of an insulin formulation where the device is associated with an adsorbent for removing stabilizing agents and/or selected compounds from the insulin formulation prior to introducing to the patient.

### Background of the Invention

Insulin and other injectable medications are commonly delivered with drug delivery pens, whereby a disposable pen needle hub is attached to the pen to facilitate drug container access and allow fluid egress from the container through the needle into the patient.

Drugs and pharmaceuticals often contain preservatives and/or stabilizing agents to extend the shelf-life of the drug or pharmaceutical. For example, insulin often contains phenol and/or m-cresol as stabilizers. These stabilizers can often produce side effects, such as irritation, inflammation, scarring and lipohypertrophy at the injection site.

Various pen needle delivery devices are known in the art for dispensing the substance to the patient. The delivery devices often use a disposable needle hub having a cannula or needle extending from a patient end of the hub for inserting into the patient. A non-patient end of the hub is coupled to the pen delivery device for delivering the substance to the patient.

The needle hub assembly is often packaged in a container containing several loose needle hubs. A needle hub is selected from the package and attached to the pen needle delivery device for injecting the patient and then removed to be discarded. The needle hub package includes an outer cover that encloses the needle hub and a removable seal that is peeled from the outer cover to open the cavity so that the needle hub can be removed. The needle hub can have threaded non-patient end that is threaded onto the delivery device. The delivery device with the attached needle hub is then removed from the outer cover. An inner needle shield is attached to the needle hub to cover the cannula until the device is ready for use. The shield is removed to expose the cannula for use to deliver the substance to the patient. After use, the needle hub can be inserted back into the outer cover to enclose the exposed cannula. The pen delivery device separates from the needle hub leaving the needle hub in the outer cover. The prior delivery devices are generally suitable for delivering the insulin directly from a storage container or supply.

Existing pen needle assemblies are disclosed in U.S. Patent Application Publication Nos. 2006/0229562 to Marsh et al. and 2007/0149924 to R. Marsh.

Although the prior devices have been suitable for the intended use, there is a continuing need in the industry for improved delivery devices to reduce the irritation and inflammation at the injection site.

US 2016/354542 A1 teaches the concept, and method of creating, a dual use device intended for persons who take insulin. In one embodiment, the device of US 2016/354542 A1 is an insulin delivery cannula, the outer wall of which contains electrodes, chemical compounds and electrical interconnects that allow continuous glucose sensing and delivery of data to a remote device. US 2016/354542 A1 describes that the main problem in attempting to sense glucose at the site of insulin delivery has been the high current resulting from oxidation by the sensor of the preservatives in the insulin formulations. According to US 2016/354542 A1, one means of eliminating these interferences is to poise the indicating electrode(s) of the sensor at a bias sufficiently low to avoid the signal from oxidation of the preservatives. In US 2016/354542 A1, one way of obtaining a glucose signal at a low bias is to use an osmium-ligand-polymer complex instead of conventional hydrogen peroxide sensing. Another is to use a size exclusion filter located in line with the insulin delivery tubing in order to remove the smaller phenolic preservative molecules while allowing the larger insulin molecules to pass unimpeded.

Further background art relevant for the present invention is described in US 2002/060180 A1 and US 2017/232204 A1.

### Summary of the Invention

The present invention is defined in claim 1.

The present invention is directed to a delivery device for delivering a drug or pharmaceutical to a patient. The delivery device is an injection or infusion device having a cannula for delivering the substance to the patient. The delivery device is particularly suitable for treating insulin formulations shortly before introducing the insulin to the patient.

The delivery device is used in conjunction with a filter and/or adsorbent that is able to remove selected substances or compounds from the medication, drug or pharmaceutical prior to delivering the medication, drug or pharmaceutical to the patient. In one embodiment, the filter or adsorbent is a separate unit that is attached to the delivery device and/or positioned in the flow path of the medication prior to introducing the medication to the patient. In other embodiments, the adsorbent is incorporated into the device where the medication passes through the adsorbent at the time of delivery to the patient.

In one embodiment, a delivery device is able to inject a substance, such as insulin, medication, drug or pharmaceutical into the patient at controlled rates and dosage, which can be over an extended period of several days. Examples of delivery devices include a pen needle, infusion set, patch pump, catheter, or other delivery device suitable for introducing a medication and particularly insulin to a patient in a unit dosage or sustained continuous delivery. The delivery device is configured so that the substance passes through a filter and/or adsorbent to remove selected compounds or materials from the substance just prior to introducing into the patient. In one embodiment, the delivery device includes an injection member, such as a needle, cannula, or catheter, where the adsorbent or filter is oriented upstream of the injection member to filter and treat the insulin, medication, drug or pharmaceutical prior to delivering to the injection member and prior to injecting to the patient.

One aspect of the delivery device provides a storage container that is able to store a selected volume of the substance prior to use and delivery to the patient. The delivery device is able to removed selected substances and compounds from a dosage amount of the substance before introducing to the patient. The delivery device in one embodiment includes a filter or adsorbent that is a separate unit from the storage container so that the substance being stored in the storage container does not contact the adsorbent until the substance is ready for delivery to the patient.

The delivery device in one embodiment is connected to a filter or adsorbent member to treat the substance as the substance is dispensed from a storage container over an extended period of time of several days and before introducing to the patient. The substance contacts the adsorbent after dispensing from the storage container to remove selected compounds from the substance and before supplying the substance to a delivery member.

In one embodiment, the delivery device includes the adsorbent positioned between the storage container and the injection member. The delivery device can be an infusion set for insulin delivery having a pump or other dispensing mechanism, a storage container for the insulin, and a catheter or cannula for delivering the insulin to the patient. The adsorbent is located in the fluid flow path upstream of the catheter to treat the insulin just before supplying to the catheter and delivering to the patient. The absorbent can be positioned as close as possible to the catheter to limit the time between the point in the flow path where the selected compounds are removed from the insulin and the time the insulin is introduced to the patient. Positioning the adsorbent close to the catheter limits the amount of treated insulin remaining in the assembly.

The delivery device is a delivery pen and the delivery member comprises a pen needle including said cannula, said cannula having a proximal end for piercing a septum at a distal end of said filter cartridge to access said internal cavity and a distal end for delivering said insulin. In an embodiment, the delivery device in the form of a pen needle assembly has a cartridge containing the insulin and a dispensing mechanism. A pen needle having a needle hub and a needle is attached to the pen needle assembly for injecting the insulin into the patient. An adsorbent is provided in the flow path between the cartridge and the pen needle to treat the insulin as the insulin is being delivered to the patient.

The delivery device in one embodiment is an insulin delivery device such as an infusion set, pen needle assembly, patch pump, catheter, or other delivery mechanism for delivering insulin to the patient in a controlled dosage. A cartridge containing the adsorbent is connected to the delivery device in the flow path of the substance being delivered to the patient. The cartridge is positioned in the insulin fluid flow path upstream of the cannula or catheter for the delivery device. Alternatively, the cartridge containing the adsorbent can be provided in a pen needle assembly where the insulin dispensed from the cartridge passes through the adsorbent and then to the pen needle before delivering to the patient. In other embodiments, the adsorbent can be incorporated in the cartridge or at the outlet of the cartridge where the insulin passes through the adsorbent as the insulin is dispensed from the cartridge to the pen needle.

The delivery device in another embodiment can be an infusion pump having a supply tube or other fluid connection extending from the pump mechanism to a catheter or cannula for positioning in the patient to deliver the insulin to the patient in a controlled and continuous delivery. A cartridge containing an adsorbent is positioned upstream of the catheter and in close proximity of the catheter or cannula so that the insulin passes through the adsorbent during delivery and infusion to the patient.

In one embodiment, the drug being delivered is an insulin formulation, such as fast acting insulin formulation, containing a stabilizing and/or preserving agent that is present to extend the shelf-life of the insulin. An example of a preservative is a stabilizing phenolic compound, such as phenol, m-cresol, and mixtures thereof. The adsorbent is able to remove at least a portion of the phenol and m-cresol from the insulin solution by passing the insulin formulation through a bed of the adsorbent or a cartridge containing the adsorbent. The absorbent is used in an amount to remove an amount of the phenolic stabilizers sufficient to reduce irritation at the injection site that is normally caused by the presence of the phenolic stabilizing agents in the insulin. The adsorbent is selective to the phenolic stabilizer, and particularly m-cresol, without reducing the effectiveness or potency of the insulin. Reducing the concentration or amount of the phenolic stabilizing agents in the insulin that contacts the tissue at the delivery site improves the absorption of the insulin at the infusion site and reduces inflammation.

In one embodiment, the adsorbent is activated carbon or activated charcoal that is able to adsorb phenol and m-cresol effectively by contacting the insulin solution with the adsorbent without reducing the effectiveness or potency of the insulin solution at the time of delivery to the patient. Acid activated charcoal, such as phosphoric acid treated activated charcoal, is particularly suitable for adsorbing and removing phenol and m-cresol from the insulin formulation without significantly lowering the potency of the insulin. Activated carbon that is chemically activated by phosphoric acid at pH 6.7 is effective in selectively removing phenol and/or m-cresol from insulin.

The adsorbent is positioned relative to the delivery or injection device to contact the insulin formulation at the time of or immediately before introducing into the patient. The adsorbent is included in an amount to provide a contact time with the insulin sufficient to remove a desired amount of the phenolic stabilizing agent from the insulin without reducing the effectiveness of the insulin. In one embodiment, the adsorbent is located at or near the injection member, such as a catheter or cannula, so that the residence time of the resulting treated insulin downstream of the adsorbent is sufficiently short to minimize degradation, denaturing, or loss of potency of the insulin delivered to the patient. The absorbent removes an amount of the phenolic compounds to reduce or inhibit the inflammation or irritation at the delivery site and improve absorption by the patient.

The objects, advantages, and features of the device will become apparent from the following detailed description, which, taken in conjunction with the annexed drawings, discloses exemplary embodiments of the invention.

### Brief Description of the Drawings

The above benefits and other advantages of the various embodiments of the present invention will be more apparent from the following detailed description of exemplary embodiments of the present invention and from the accompanying figures, in which:
Fig. 1 is an elevational view of a pen needle assembly according to the invention;
Fig. 2 is a cross sectional view of the pen needle of Fig. 1;
Fig. 3 is a cross sectional view of the cartridge of Fig. 1;
Fig. 4 is a cross sectional view of the pen needle including the adsorbent;
Fig. 5 is a top view of a catheter assembly in an embodiment;
Fig. 6 is a side view of the catheter assembly of Fig. 5;
Fig. 7 is a cross sectional view of the cartridge of Fig. 5;
Fig. 8 is a perspective view of an infusion set in another embodiment;
Fig. 9 is an exploded view of an infusion set in a further embodiment;
Fig. 10 is a cross sectional view of the cartridge of Fig. 9;
Fig. 11 is an exploded view of a patch pump in another embodiment;
Fig. 12 is a Table showing the amount of m-cresol remaining after treating with 15 mg of the adsorbent of basal samples;
Fig. 13 is a Table showing the amount of m-cresol remaining after treating with the adsorbent of bolus samples;
Fig. 14 is a Table showing the amount of m-cresol remaining after treating with 2.5 mg of the adsorbent of basal samples;
Fig. 15 is a Table showing the amount of m-cresol remaining after treating with the adsorbent of bolus samples; and

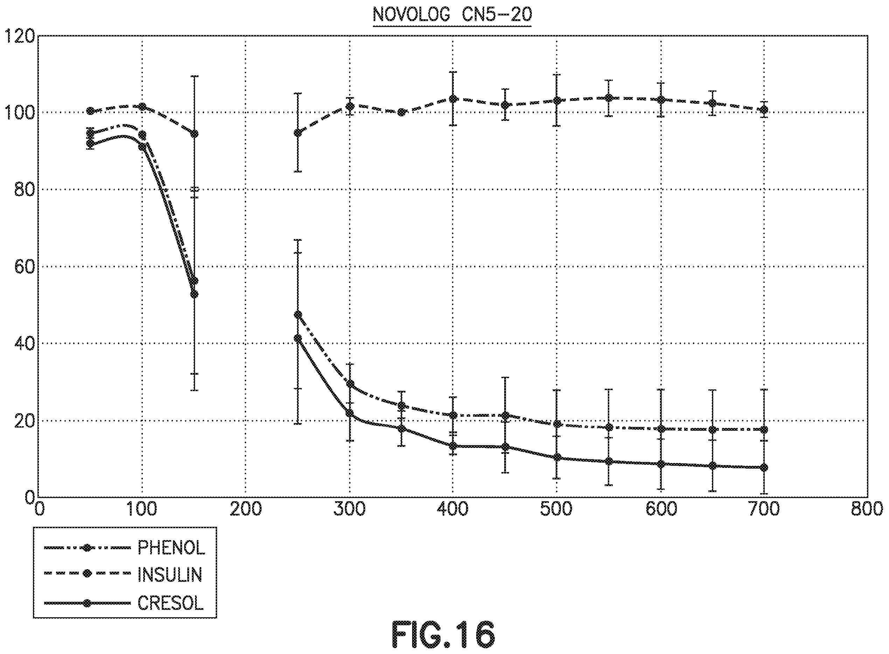

Fig. 16 is a Table showing the removal of phenol and m-cresol from a sample and the amount of insulin in the sample.

Throughout the drawings, like reference numbers will be understood to refer to like parts, components, and structures.

### Detailed Description of the Exemplary Embodiments

The present invention is directed to a delivery device and method of delivering a substance, such as insulin, medication, or a drug to a patient, and removing selected compounds or substances from the insulin, medication or drug prior to delivering to the patient.

The delivery device and method are particularly suitable for use in delivering insulin that contains a stabilizer or preservative where at least a portion of the stabilizer or preservative is removed from the insulin before delivering to the patient. The delivery device for introducing an insulin formulation into the patient is used in association with an adsorbent material that contacts the insulin formulation before introducing to the patient. In one embodiment, a method of reducing or minimizing the irritation and inflammation at the delivery or an injection site is attained by reducing the amount of the stabilizer, such as phenol and/or m-cresol in an insulin formulation before introducing to the patient. The insulin is treated with an adsorbent to remove at least a portion of the phenol and/or m-cresol where the treated insulin is introduced into the patient within a period of time to prevent denaturing or loss of insulin potency.

Reference is made to embodiments of the present invention, which are illustrated in the accompanying drawings, wherein like reference numerals refer to like elements throughout. The embodiments described herein exemplify, but do not limit, the present invention by referring to the drawings. The exemplary embodiments are presented in separate descriptions, although the individual features and construction of these embodiments can be combined in any number of ways to meet the therapeutic needs of the user.

This disclosure is not limited in its application to the details of construction and the arrangement of components set forth in the following description or illustrated in the drawings. The embodiments herein are capable of being modified, practiced or carried out in various ways. Also, it will be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely.

The delivery device and method are provided for delivering the substance, such as insulin, drug, pharmaceutical or other substance to a patient by a bolus flow delivery or basal delivery. In one embodiment, the drug is an insulin formulation or solution that is delivered to the patient in a selected and controlled dosage at an injection or infusion site.

The insulin formulation is typically a solution containing a preservative and stabilizing agent to extend the shelf-life of the insulin solution until ready for use. The stabilizing agent in one embodiment is phenol, m-cresol and mixtures thereof. The majority of Type 1 diabetics manage the condition by multiple daily injections of insulin. The daily injections result in side effects including irritation, inflammation, scarring, and lipohypertrophy and the accumulation of subcutaneous fat at the insulin injection site or infusion site. The presence of phenol and m-cresol in insulin formulation is effective as a bacteriostat and for stabilizing the insulin formulation. However, the presence of the phenol and m-cresol in the insulin with repeating or sustained injection at an injection site or infusion site can cause inflammation and irritation to the patient and can reduce insulin absorption at the site.

The phenolic excipients m-cresol and phenol present in insulin analog formulations as a bacteriostatic and stabilizing factor are cytotoxic in an in vitro system and contribute to adverse tissue reactions when delivered locally at formulation concentrations. The adverse tissue reactions result in increased pro-inflammatory cytokine levels and altered subcutaneous insulin pharmacokinetics. The deleterious reactions are dose-dependent so that as more excipient is delivered, such as in insulin infusion devices, pharmacokinetics are increasingly altered relative to initial values. Test data suggests that excipient-induced models of inflammation negatively affect the route of insulin administration and absorption. This can lead to issues of inadequate adherence.

One feature of the delivery device and method is to remove the phenolic excipients selectively from insulin formulations without interfering with the effectiveness of the insulin upon delivery to the patient. Experiments using activated charcoal as a adsorbent show the effective removal of the phenol and m-cresol from insulin formulation while maintaining the effluent insulin at formulation concentrations. The resulting treated insulin having a reduced concentration of the phenolic excipient is delivered to the patient within a period of time where substantially no denaturing or loss of potency of the insulin occurs. In one embodiment, the adsorbent is selected to remove only the phenolic excipients.

An adsorbent is used with the delivery device for removing at least a portion of the stabilizing agents, and particularly for removing at least a portion of the phenolic stabilizing agents from an insulin formulation prior to introducing to the patient. The adsorbent in one embodiment is provided in a chamber, container, or cartridge connected to or positioned in the flow path of the insulin formulation as the formulation is being supplied to the delivery or injection device. In other embodiments, the adsorbent is incorporated within the delivery device and positioned where the insulin formulation contacts the adsorbent as the insulin formulation is being delivered to the patient.

In one embodiment, the adsorbent is activated charcoal that can be in a granular, extruded, or powder form to provide a contact surface area for the insulin that is sufficient to remove an selected amount of the phenolic stabilizers to inhibit inflammation at the delivery site without denaturing or loss of potency of the insulin at the time of delivery to the patient. In the present description of the device, the terms activated charcoal and activated carbon are used interchangeably. Acid treated activated charcoal, such as phosphoric acid activated carbon, is particularly suitable for removing phenol and m-cresol from insulin formulations. In one embodiment, the activated charcoal is a chemically active carbon obtained by treatment with phosphoric acid. The activation can be by phosphoric acid at pH 6.7. Commercially available phosphoric acid treated activated charcoal can be used for the removal of phenol and m-cresol from insulin formulations. An example of a commercially available acid treated activated charcoal is available under the trade name CN5-20 by Cabot Corporation. The activated charcoal has a surface area to provide sufficient contact with the insulin to remove an amount of the phenolic compounds sufficient to minimize irritation and inflammation at the injection site.

The activated charcoal can be obtained from a variety of carbon sources including, for example, wood, coconut shell, olive pits, peat, lignite, coal or other suitable carbon source. The activation in one embodiment is by chemical activation with phosphoric acid to provide the beneficial porosity, pore volume, surface area, surface chemistry, and pore size distribution. The activated charcoal typically has a surface area of greater than 1,000 m²/g. The activated charcoal can have a pore volume of about 0.26-1.16 cm³/g, and generally about 0.40-0.70 cm³/g. In other embodiments, the activated charcoal can have a surface area of 1500 m²/g or greater. In further embodiments, the activated charcoal can have surface area of greater than 2300 m²/g and in some circumstances a surface area of greater than 3,000 m²/g depending on the method of activation.

The adsorbent is present in an amount to provide a contact time with the insulin formulation that is sufficient to remove a desired amount of the phenol, m-cresol or other stabilizing agents contained in the insulin formulation to reduce irritation and inflammation at the injection site without denaturing and without reducing the potency of the insulin. The adsorbent is located in the flow path of the insulin formulation as close to the injection member or delivery site as reasonably possible to limit degradation of the insulin formulation before introducing to the patient.

The amount of activated charcoal in the assembly complements the dosage, and flow rate of the insulin depending on the delivery by basal flow or bolus flow delivery to provide the desired rate of adsorption of the phenolic stabilizers. In one embodiment, the amount of the adsorbent provides removal of about 95% of the m-cresol after 4 days and about 60% after 7 days at a basal flow rate.

In the embodiment shown, the adsorbent can be enclosed in a container or cartridge that is separate from the delivery or injection device. In other embodiments, the adsorbent can be enclosed in a chamber within or as a component of the delivery device or in a supply container or cartridge that treats the insulin formulation as the formulation is dispensed from the supply container.

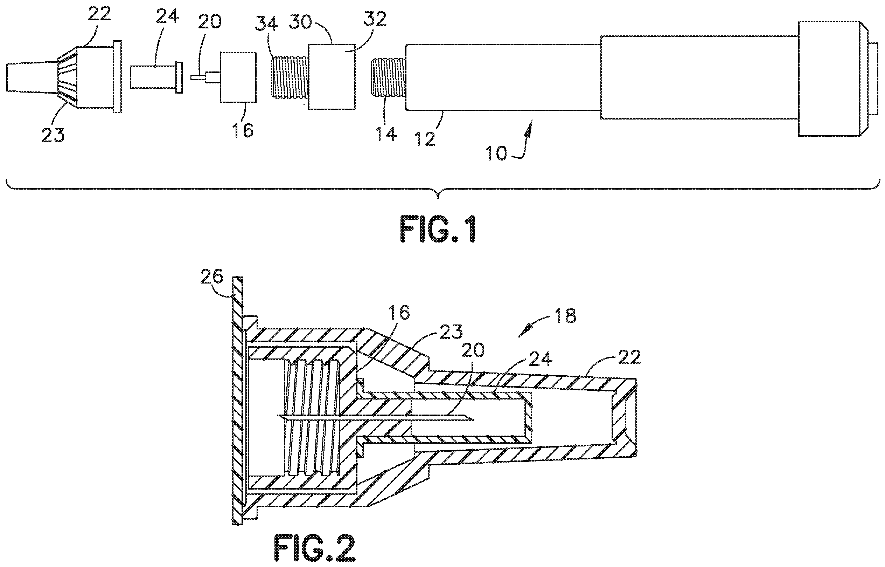

In one embodiment the delivery device is a pen needle delivery device 10, as shown in Fig. 1, which typically comprises a dose knob/button, an outer sleeve 12, and a cap. A dose knob/button allows a user to set the dosage of medication to be injected. The outer sleeve 12 is gripped by the user when injecting medication. The cap is used by the user to securely hold the pen needle device 10 in a shirt pocket or other suitable location and provide cover/protection from accidental needle injury.

In standard pen needle devices the dosing and delivery mechanisms are found within the outer sleeve 12 and is not described in greater detail here as they are understood by those knowledgeable of the art. A medicament cartridge is typically attached to a standard pen injector housing by known attachment mechanism. The distal movement of a plunger or stopper within the medicament cartridge causes medication to be forced into the reservoir housing. The medicament cartridge is sealed by a septum and punctured by a septum penetrating needle cannula located within a reservoir or housing. Reservoir housing is preferably screwed onto the medicament cartridge although other attachment mechanism can be used. The pen needle delivery device can be a standard pen delivery device known in the industry so that the pen needle delivery device is not shown in detail. The pen needle assembly 18 as shown in Fig. 2 includes a needle hub 16 supporting a cannula 20, an outer cover 22, and an inner shield 24. A protective seal 26 is attached to the open end of the outer cover as shown in Fig. 2 to enclose the needle hub and cannula to maintain a clean and sterile condition. The seal 26 can be a label or other closure member that can be easily peeled from the outer cover to access the needle hub during use.

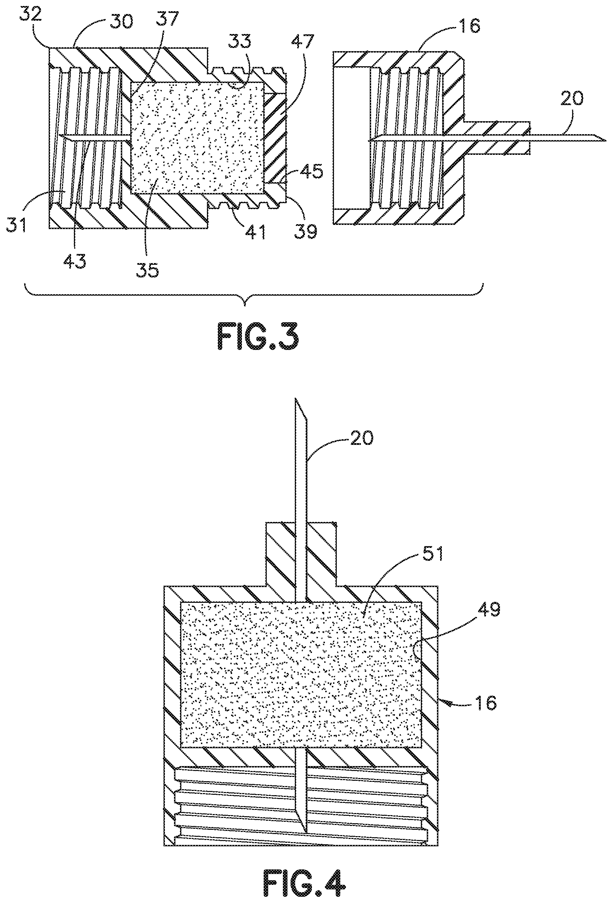

In the embodiment shown, the pen needle delivery device 10 is provided with an adsorbent, such as activated charcoal, to treat the insulin before delivery to the patient. In one embodiment, a filter cartridge 30 is provided in or on the outlet of the pen needle delivery device as shown in Fig. 1. The filter cartridge 30 has an inlet end 32 for connecting to the delivery pen 10 whereby the insulin formulation passes through the cartridge 30 to an outlet end 34. The inlet end 32 of the cartridge 30 has open end with internal threads 31 for connecting with the delivery pen shown in Fig. 3. The outlet end 34 is configured with external threads 41 for coupling with the pen needle hub 16 for introducing the treated insulin to the patient. In the embodiment shown, the outlet end 34 has a threaded coupling for mating with the internal threads of the needle hub 16.

As shown in Fig. 3, the cartridge 30 has an internal cavity 33 that contains the adsorbent 35 in an amount to provide sufficient contact time with the insulin to remove a desired amount of the stabilizers and particularly phenolic stabilizer compounds. The cartridge has a shape and internal volume to provide a residence time for the insulin to remove the stabilizer compounds and limit a retention volume after the injection to minimize denaturing of the insulin remaining in the cartridge. The internal cavity 33 is formed by a bottom wall 37 and a top wall 39. Bottom wall 37 supports a cannula 43 for piercing a septum in the delivery pen to carry the insulin from the supply reservoir or cartridge of the delivery pen to the cavity containing the adsorbent 35. The top wall 39 in the embodiment shown has an opening 45 with a septum 47 for receiving the non-patient end of the needle 20 of the pen needle 16 to provide the fluid communication between the insulin passing through the cartridge and the pen needle 16 for delivery to the patient. In the embodiment shown, the bottom wall and top wall of the cartridge are integrally formed with the body of the cartridge. In other embodiments, the bottom wall and top wall are formed as separate components and attached to the body of the cartridge. The cartridge can be constructed for single use for disposal after use or for multiple use for a predetermined time while the adsorbent is effective in removing the stabilizers from the insulin without denaturing or loss of potency of the insulin.

In one embodiment, the cartridge is transparent or has a viewing portion or window having sufficient transparency to enable the user to observe the contents of the cartridge before and after use. The transparent portion of the cartridge is located in a position that the user is able to visualize fibrillation or changes in the insulin caused be denaturing after the stabilizing agents are adsorbed and removed by the adsorbent.

The cartridge 30 has a volume of the adsorbent to contact a dosage of the insulin formulation and remove a selected amount of the phenol, m-cresol or other stabilizing agent from the insulin formulation before introducing to the patient. The cartridge 30 typically has a volume of the activated charcoal to remove an amount of phenol and/or m-cresol from the insulin formulation at typical flow rates of the pen needle delivery assembly 10 to inhibit inflammation and irritation at the injection site. In other embodiments, the adsorbent is contained in a chamber of the cartridge positioned within the sleeve 12 of the pen needle delivery device where the insulin formulation from the cartridge passes through the adsorbent as the insulin is supplied to the pen needle 18.

In the embodiment shown, the cartridge 30 is positioned relative to the pen needle to minimize the time before the treated insulin with the reduced phenol and/or m-cresol concentration is introduced to the patient to minimize the loss of insulin potency by degradation or denaturing of the insulin.

The cartridge 30 in the embodiment of Fig. 3 is a separate unit that is coupled to the pen needle 16 and the delivery pen 10. In another embodiment shown in Fig. 4, the pen needle 16 includes an internal chamber or cavity 49 containing the adsorbent 51. The needle 20 extends from the distal end and communicates with the cavity 49. A threaded proximal end has a needle for piercing the septum of the delivery pen in a manner similar to a typical pen needle.

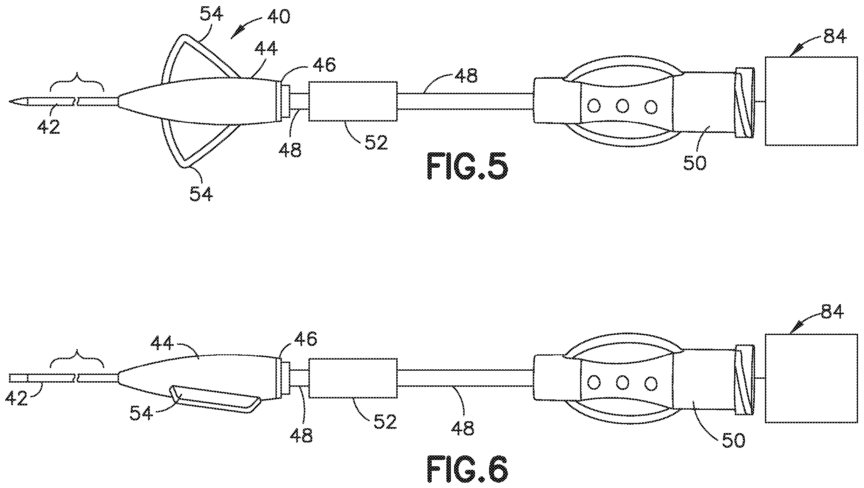

In the embodiment of Figs. 5-7, the delivery device is an intravenous catheter assembly 40 including a catheter 42 connected to a catheter adapter 44. The catheter adapter 44 has a proximal end 46 for connecting to a fluid supply tube 48. The fluid supply tube 48 includes a coupling 50, such as a luer fitting or other threaded coupling, for connecting to a dispensing member 84 such as an infusion pump. In other embodiments, the coupling 50 can be a friction fit or interference fit that provides a fluid tight fit. The dispensing member 84 is connected to or contains an insulin supply.

In the embodiment shown, the catheter adapter 44 includes winged extensions 54 that project outwardly from the body of the catheter adapter 44. The winged extensions 54 include an adhesive for attaching the catheter adapter to the patient to maintain a desired position of the catheter adapter following catheterization.

As shown in Figures 5-7, a cartridge 52 is positioned in the supply tube 48 upstream of the catheter adapter 44 and downstream of the insulin supply. The cartridge 52 contains the adsorbent, such as the activated charcoal, so that the treated insulin formulation dispensed through the supply tube 48 contacts the adsorbent before supplying to the catheter 42 and introducing into the patient. Cartridge 52 in the embodiment shown is positioned as close to the catheter adapter 44 as reasonably possible to minimize the residence time of the treated insulin formulation between the time the treated insulin formulation exits the cartridge 52 and the time the treated insulin formulation is delivered to the patient. The cartridge 52 can be made of a suitable glass or plastic that can be clear or opaque. In one embodiment, the container is sufficiently transparent to detect and observe the occurrence of insulin fibrillation by visual inspection.

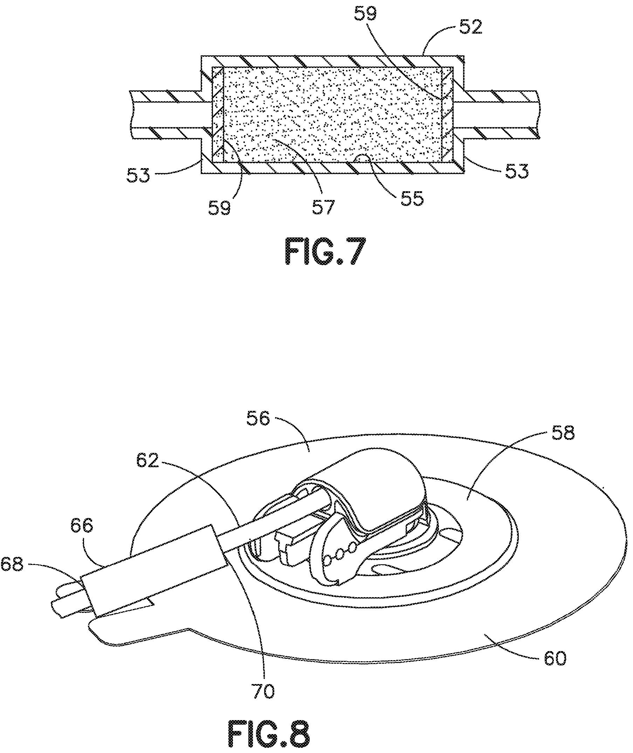

As shown in Fig. 7, the cartridge 52 has a body with end walls 53 with an opening connected to the supply tube 48 and an internal cavity 55 enclosing the adsorbent 57. In the embodiment shown, a filter or porous member 59 is positioned at the open ends of the cartridge to retain the adsorbent 57 within the cartridge 52. The porous member 59 has pore size corresponding to the particle size of the adsorbent to retain the adsorbent in the cartridge within inhibiting the flow of insulin through the adsorbent and delivering the adsorbent to the catheter 42. The catheter assembly is generally constructed as unit for single use so that the entire assembly is discarded after use. Alternatively, the cartridge 52 can have suitable couplings at each end for coupling with complementing couplings on the tube 48 for replacement after use to allow replacement or replenishment of the adsorbent while the catheter is positioned in the patient.

Another embodiment as shown in Figs. 8-10, the delivery device is an infusion set 56 as known in the art. The infusion set 56 includes a base 58 and a flexible pad 60 having an adhesive for attaching the infusion set to the patient. The base 58 supports a flexible cannula or flexible catheter and insertion needle as known in the art for delivering the insulin formulation to the patient for an extended period of time. An example of an insertion needle and catheter are disclosed in US Patent Publication No. 2017/0028128. The infusion set typically provides the insulin delivery for several days in the same infusion site to provide basal flow to the patient. The cannula or catheter as known in the art is generally a solve, flexible cannula or catheter that is positioned in the patient to provide a controlled delivery of the insulin to the patient.

A fluid supply tube 62 is connected to a removable fluid coupling 64 for connecting to the base 58 to supply the insulin formulation to the catheter. A cartridge 66 is connected to the supply tube 62 so that the insulin formulation passes through the cartridge before delivering to the infusion set 56. The cartridge 66 has an inlet 68 that receives the insulin formulation from a supply and pump mechanism, and an outlet 70 for directing the treated insulin formulation to the catheter.

The cartridge 66 contains an amount of the adsorbent, such as activated charcoal, to contact the insulin formulation and remove at least a portion of the phenol, m-cresol or other stabilizing agents from the insulin formulation before discharging through the outlet 70 of the cartridge. The cartridge 66 can be a single component that can be connected to supply tube 62 by couplings so that the cartridge 66 can be removed when the adsorbent reaches the end of its useful life. A replacement cartridge can then be connected to the supply tube 62 for continued delivery of a treated insulin to the patient.

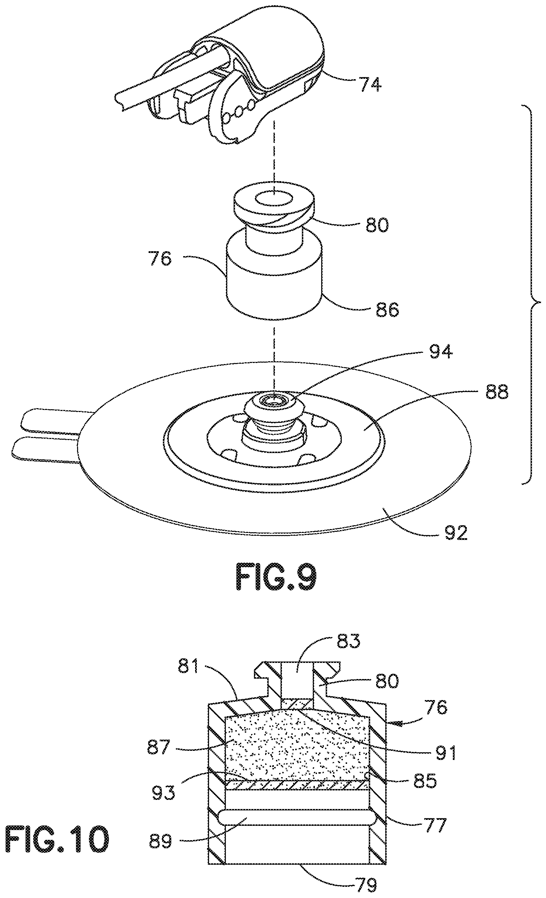

In a further embodiment shown in Fig. 9, an infusion set 72 having a fluid coupling 74 is connected to an adsorbent cartridge 76. As in the previous embodiment, the infusion set includes a base 88 that supports a fluid coupling 94, and a flexible adhesive pad 92. A catheter or cannula extends from the base 88 for delivering the insulin to the patient. The catheter or cannula in the infusion set is generally a soft, flexible cannula as commonly used in an infusion set. The cartridge 76 in the embodiment shown has a suitable coupling mechanism for connecting to the fluid coupling 74 and providing a fluid tight seal. An inlet end of cartridge 76 includes a coupling 80 for connecting to the fluid coupling 82 of the dispensing device or pump mechanism for supplying insulin formulation to the infusion set.

As shown in Fig. 10, the cartridge 76 has a side wall 77, an open bottom end 79 and, a top wall 81. The top wall 81 supports the coupling 80. The coupling 80 has an internal passage 83 for fluid communication with the cavity 85 of the cartridge for containing the adsorbent 87. The open bottom end has a recess 89 for coupling with the coupling 94 of the infusion set.

The passage 83 includes a porous membrane, screen, or filter 91 and the open bottom end includes a porous membrane, screen, or filter 93 to form the cavity 85 and retain the adsorbent 87 within the cavity 85. The porous membranes have pore size to retain the adsorbent in the cavity while allowing sufficient insulin flow through the cartridge to the catheter of the infusion set.

The activated charcoal as an adsorbent has been found to be effective in filtering and removing phenol from insulin lispro, such as sold under the tradename Humalog over a 7 day period. The activated charcoal has also been found to be effective in removing phenol and m-cresol from insulin aspart, such as sold under the tradename Novolog. No aggregation or fibrillation of insulin was observed for either of these formulations during or after contact with the activated charcoal over a 7 day.

The activated adsorbent is particularly suitable for treating insulin in an infusion device such as the infusion devices of Figs. 8-10 where the catheter is positioned in the patient for several days to provide a continuous and/or controlled delivery of the insulin formulation. The treatment of the insulin formulation to remove the phenol and m-cresol from the insulin formulation just prior to introducing to the patient effectively reduces or inhibits irritation and pain at the infusion site and improves the absorption of insulin over a period of several days at the infusion site.

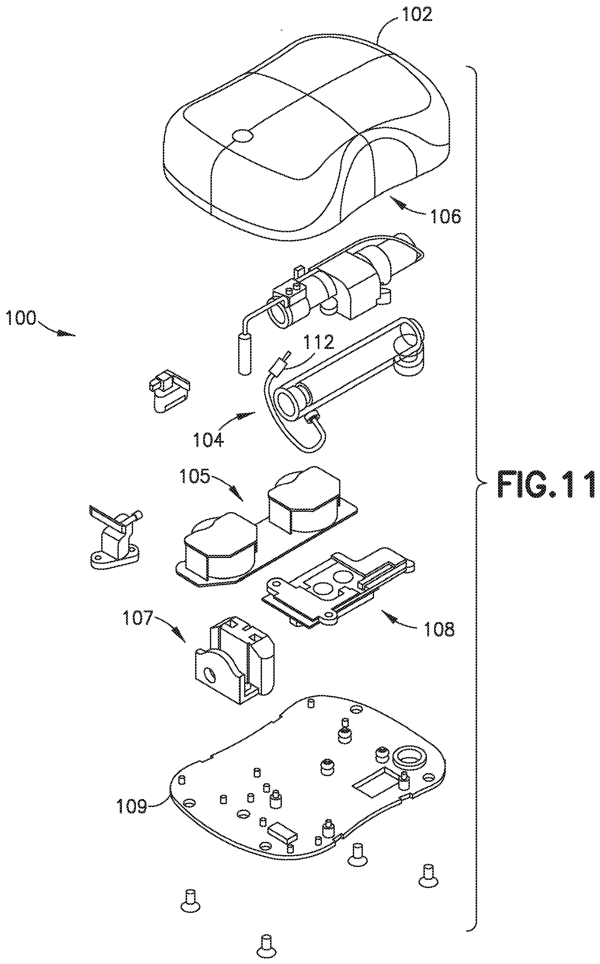

Fig. 11 shows another embodiment of the device where the delivery device is a patch pump 100. Fig. 11 is an exemplary embodiment of a patch pump 100. The patch pump 100 is illustrated with a see-through cover for clarity and illustrates various components that are assembled to form the patch pump 100. Fig. 11 is an exploded view of the various components of the patch pump, illustrated with a solid cover 102. The various components of the patch pump 100 may include: a reservoir 104 for storing insulin; a pump 103 for pumping insulin out of the reservoir 104; a power source 105 in the form of one or more batteries; and an insertion mechanism 107 for inserting an inserter needle with a catheter into a user's skin. Control electronics 108 are included in the form of a circuit board with optional communications capabilities to outside devices such as a remote controller and computer, including a smart phone. A dose button 106 on the cover 102 is included for actuating an insulin dose, including a bolus dose. A base 109 to which various components above may be attached by fasteners 110. The patch pump 100 also includes various fluid connector lines that transfer insulin pumped out of the reservoir 104 to the infusion site. An example of a patch pump having a catheter and insertion mechanism is disclosed in US Patent Publication No. 2017/0028128.

In the embodiment shown, the patch pump 100 includes a cartridge 112 connected to the fluid supply tube from the reservoir 104 so that the insulin from the reservoir passes through the cartridge 112 before catheter. In the embodiment shown, cartridge 112 is constructed in a manner similar to the embodiment of Fig. 7 and is unitary part of the assembly of the patch pump for single use. The reservoir and cartridge in other embodiments, can be a removable unit for replacement in the patch pump.

### Example 1

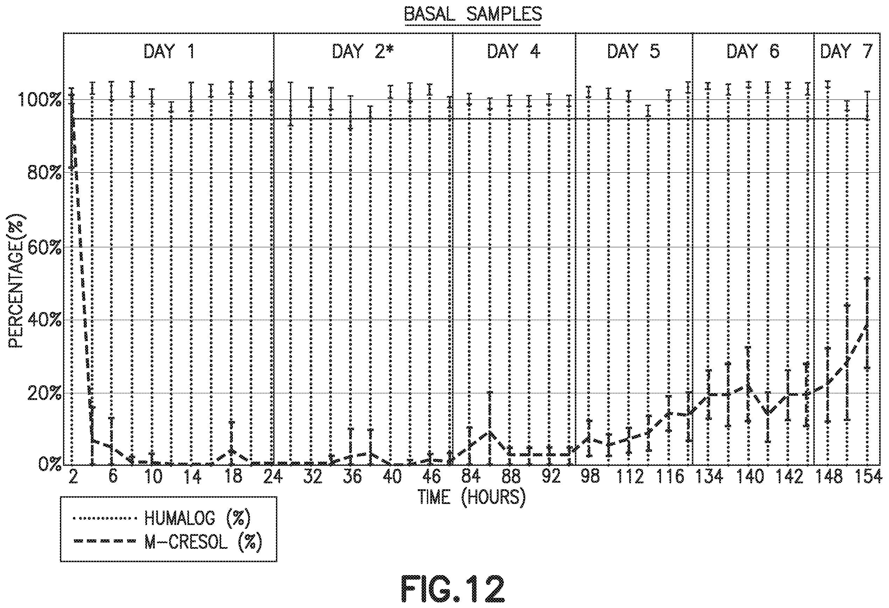

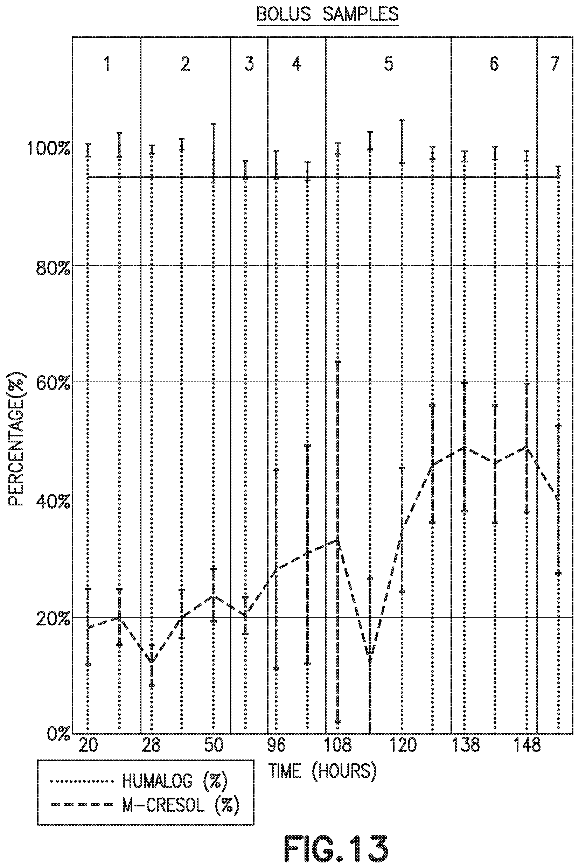

The effectiveness of the activated charcoal for removing phenol and m-cresol from insulin formulations was tested using a known infusion formulation. The insulin formulation obtained under the tradename Humalog containing m-cresol as a stabilizer was passed through a bed of 15 mg of acid-treated activated charcoal for a period of 7 days. The acid-treated activated charcoal was obtained under the tradename CN5-20 from Cabot Corporation. The insulin formulation was passed through the activated charcoal at a rate corresponding to a basal flow delivery of insulin and a bolus flow delivery of insulin. As shown in the table of Fig. 12 representing the basal flow, the activated charcoal was effective in removing about 95% by weight of m-cresol through day 4 and about 60% by weight through day 7. The insulin potency was maintained at greater than 93% through day 7. The bolus flow as shown in the table of Fig. 12 shows about 60% m-cresol removed after day 7, which corresponds to about 40% by weight of m-cresol remaining after day 7 with substantially small losses of insulin potency similar to the basal flow of Figure 12. The tables of Figs. 12 and 13 show that the basal flow rate was about 20% more effective in removing m-cresol than bolus flow rates.

### Example 2

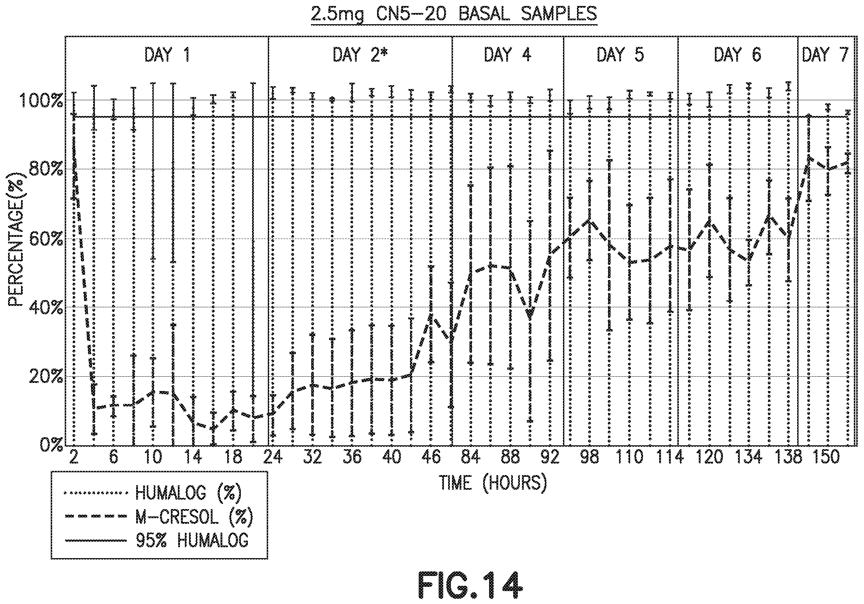

Example 2 was performed in a similar manner as in Example 1 except for the use of 2.5mg of the acid-treated activated charcoal with a similar insulin volume. As shown in the basal flow of the table in Fig. 13, the amount of the activated charcoal was less effective in removing m-cresol from the insulin formulation at the same flow rates and volumes. The table of Fig. 13 shows that after day 7 the activated charcoal was effective in removing about 80% by weight of the m-cresol present in the insulin. The table of Fig. 10 shows the effectiveness of the removal of m-cresol for the bolus flow. As shown in Fig. 14, the amount of the activated charcoal removed about 25% by weight of the m-cresol after day 7. The results of Example 1 and Example 2 demonstrate the correlation between the volume of the insulin formulation, the amount of the activated charcoal, and the length of time of contact of insulin with the activated charcoal, and the effective removal of the m-cresol from the insulin formulation.

### Example 3

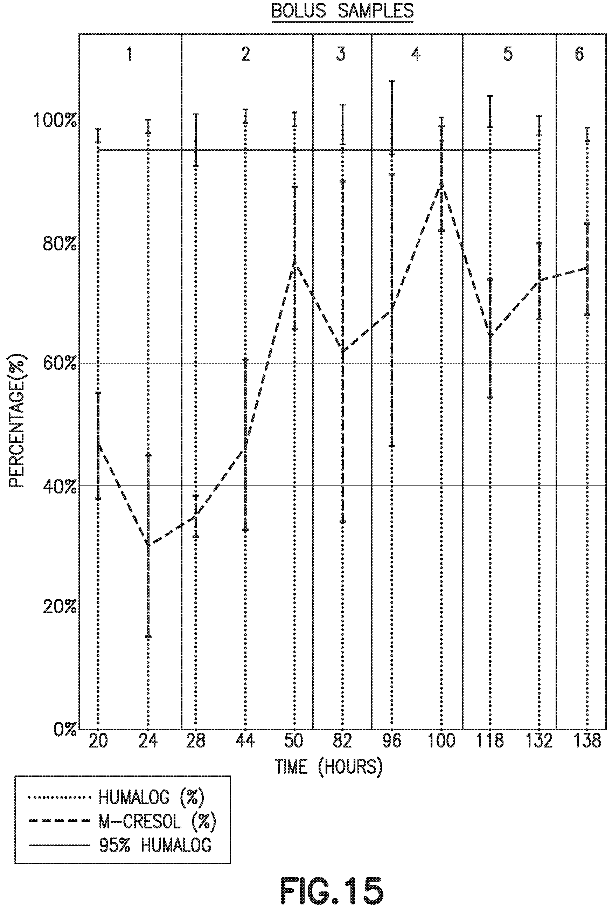

In this example, the insulin formulation was obtained under the tradename Novolog and tested with 15 mg of acid-treated activated charcoal by the tradename CN5-20. The activated charcoal was shown to effectively remover m-cresol and phenol from the insulin formulation as shown in the table in Fig. 15 without reducing the potency of the insulin.

The activated charcoal is found to be effective in removing phenol and m-cresol from insulin formulations immediately before introducing to the patient. The activated charcoal is effective in removing the phenol and m-cresol from insulin formulations at typical flow rates of infusion devices that provide a controlled and/or continuous insulin delivery without loss of insulin potency. The reduced content of the phenol and m-cresol from the insulin formulation reduces the irritation and inflammation at the injection site and improves adsorption of insulin at the injection site over a prolonged period of time.

The foregoing embodiments and advantages are merely exemplary and are not to be construed as limiting the scope of the present invention. The description of an exemplary embodiment of the present invention is intended to be illustrative, and not to limit the scope of the present invention. Various modifications, alternatives, and variations will be apparent to those of ordinary skill in the art, and are intended to fall within the scope of the invention. It is particularly noted that the features of different embodiments and claims may be combined with each other as long as they do not contradict each other. Accordingly, all such modifications are intended to be included within the scope of this invention as defined in the appended claims and their equivalents.

## Claims

1. A delivery device for delivering insulin to a patient, comprising:
a storage container (12) containing the insulin;
a delivery member (16) comprising a cannula (20) and connected to said storage container by a fluid pathway for injecting the insulin into the patient; and
a filter cartridge (30) having an inlet end (32) connected to the storage container, an outlet end (34) connected to the delivery member, and an internal cavity (33), an activated charcoal adsorbent positioned in said internal cavity and in the fluid pathway between said storage container and said delivery member for removing selected compounds from said insulin before delivering to the patient;
**characterized in that**
the delivery device is a delivery pen and the delivery member comprises a pen needle including said cannula, said cannula having a proximal end for piercing a septum (47) at a distal end of said filter cartridge (30) to access said internal cavity and a distal end for delivering said insulin.

2. The delivery device of claim 1, wherein said filter cartridge (30) has a cannula (43) for piercing a septum of the delivery device (10).

3. The delivery device of claim 1, wherein said activated charcoal adsorbent is positioned for removing a phenolic stabilizing agent from said insulin formulation before delivering to the patient.

4. The delivery device of claim 1, wherein said activated charcoal adsorbent has a residence time in said delivery member to obtain substantially no denaturing or loss of efficacy before injecting into the patient.

5. The delivery device of claim 3, wherein said activated charcoal adsorbent comprises a phosphoric acid treated activated charcoal adsorbent.

6. The delivery device of claim 5 where said filter cartridge has a configuration to provide a contact time of the insulin with said activated charcoal to remove a predetermined amount of phenol and/or m-cresol from the insulin.

7. The delivery device of claim 1, wherein said insulin contains a phenolic stabilizing agent, and said delivery device directs said insulin through said fluid pathway, and where said activated charcoal adsorbent is included in an amount to remove at least about 60% by weight of the phenolic stabilizing agent from said insulin formulation over a period of time of at least four days while maintaining an insulin potency of at least about 73% relative to untreated insulin

8. The delivery device of claim 7, wherein said delivery device is configured for a continuous and controlled delivery of said insulin for a predetermined period of time, and where said activated charcoal adsorbent is included in an amount to remove at least about 60% by weight of the phenolic stabilizing agent from said insulin formulation, and said insulin formulation delivered from said infusion set exhibiting an insulin potency of at least about 73% after about 7 days.

9. The delivery device of claim 7, wherein said phenolic stabilizing agent is selected from the group consisting of phenol, m-cresol, and mixtures thereof.

10. The delivery device of claim 1 for delivering insulin, where said insulin includes a phenolic stabilizing agent in an amount to stabilize said insulin.

11. The delivery device of claim 10, wherein said activated charcoal comprises a phosphoric acid activated charcoal.

## Patentansprüche

1. Abgabevorrichtung zur Abgabe von Insulin an einen Patienten, die aufweist:
einen Speicherbehälter (12), der das Insulin enthält;
ein Abgabeelement (16), das eine Kanüle (20) aufweist und mit dem Speicherbehälter durch einen Fluidweg zur Injektion des Insulins in den Patienten verbunden ist; und
eine Filterkartusche (30) mit einem Einlassende (32), das mit dem Speicherbehälter verbunden ist, einem Auslassende (34), das mit dem Abgabeelement verbunden ist, und einem Innenhohlraum (33), wobei ein Aktivkohle-Adsorptionsmittel in dem Innenhohlraum und in dem Fluidweg zwischen dem Speicherbehälter und dem Abgabeelement positioniert ist, um vor der Abgabe an den Patienten ausgewählte Verbindungen aus dem Insulin zu entfernen;
**dadurch gekennzeichnet, dass**
die Abgabevorrichtung ein Abgabe-Pen ist und das Abgabeelement eine Pen-Nadel aufweist, welche die Kanüle aufweist, wobei die Kanüle ein proximales Ende zum Durchstechen eines Septums (47) an einem distalen Ende der Filterkartusche (30) aufweist, um auf den Innenhohlraum und ein distales Ende zur Abgabe des Insulins zuzugreifen.

2. Abgabevorrichtung nach Anspruch 1, wobei die Filterkartusche (30) eine Kanüle (43) zum Durchstechen eines Septums der Abgabevorrichtung (10) aufweist.

3. Abgabevorrichtung nach Anspruch 1, wobei das Aktivkohle-Adsorptionsmittel positioniert ist, um ein phenolisches Stabilisierungsmittel vor der Abgabe an den Patienten aus der Insulinformulierung zu entfernen.

4. Abgabevorrichtung nach Anspruch 1, wobei das Aktivkohle-Adsorptionsmittel eine Verweildauer in dem Abgabeelement hat, um vor der Injektion in den Patienten im Wesentlichen keine Denaturierung oder keinen Wirksamkeitsverlust zu erfahren.

5. Abgabevorrichtung nach Anspruch 3, wobei das Aktivkohle-Adsorptionsmittel ein mit Phosphorsäure behandeltes Aktivkohle-Adsorptionsmittel enthält.

6. Abgabevorrichtung nach Anspruch 5, wobei die Filterkartusche eine Konfiguration zur Bereitstellung einer Kontaktzeit des Insulins mit der Aktivkohle aufweist, um eine vorbestimmte Menge an Phenol und/oder m-Kresol aus dem Insulin zu entfernen.

7. Abgabevorrichtung nach Anspruch 1, wobei das Insulin ein phenolisches Stabilisierungsmittel enthält, und wobei die Abgabevorrichtung das Insulin durch den Fluidweg leitet, und wobei das Aktivkohle-Adsorptionsmittel in einer Menge enthalten ist, um mindestens etwa 60 Gew.-% des phenolischen Stabilisierungsmittels aus der Insulinformulierung über einen Zeitraum von mindestens vier Tagen zu entfernen und gleichzeitig eine Insulinpotenz von mindestens etwa 73 % relativ zu unbehandeltem Insult aufrechtzuerhalten.

8. Abgabevorrichtung nach Anspruch 7, wobei die Abgabevorrichtung für eine kontinuierliche und kontrollierte Abgabe des Insulins für einen vorbestimmten Zeitraum ausgebildet ist, und wobei das Aktivkohle-Adsorptionsmittel in einer Menge enthalten ist, um mindestens etwa 60 Gew.-% des phenolischen Stabilisierungsmittels aus der Insulinformulierung zu entfernen, und wobei die aus dem Infusionsset abgegebene Insulinformulierung eine Insulinpotenz von mindestens etwa 73 % nach etwa 7 Tagen aufweist.

9. Abgabevorrichtung nach Anspruch 7, wobei das phenolische Stabilisierungsmittel ausgewählt wird aus der Gruppe bestehend aus Phenol, m-Kresol und Mischungen davon.

10. Abgabevorrichtung nach Anspruch 1 zur Abgabe von Insulin, wobei das Insulin ein phenolisches Stabilisierungsmittel in einer Menge zur Stabilisierung des Insulins aufweist.

11. Abgabevorrichtung nach Anspruch 10, wobei die Aktivkohle eine Phosphorsäure-Aktivkohle enthält.

## Revendications

1. Dispositif d'administration pour administrer de l'insuline à un patient, comprenant :
un récipient de stockage (12) contenant l'insuline ;
un élément d'administration (16) comprenant une canule (20) et relié audit récipient de stockage par un trajet de fluide pour injecter l'insuline au patient ; et
une cartouche filtrante (30) ayant une extrémité d'entrée (32) reliée au récipient de stockage, une extrémité de sortie (34) reliée à l'élément d'administration, et une cavité interne (33), un adsorbant à charbon actif positionné dans ladite cavité interne et dans le trajet de fluide entre ledit récipient de stockage et ledit élément d'administration pour retirer des composés sélectionnés de ladite insuline avant l'administration au patient ;
**caractérisé en ce que**
le dispositif d'administration est un stylo d'administration et l'élément d'administration comprend une aiguille de stylo comprenant ladite canule, ladite canule ayant une extrémité proximale pour percer un septum (47) au niveau d'une extrémité distale de ladite cartouche filtrante (30) afin d'accéder à ladite cavité interne et une extrémité distale pour administrer ladite insuline.

2. Dispositif d'administration selon la revendication 1, dans lequel ladite cartouche filtrante (30) a une canule (43) pour percer un septum du dispositif d'administration (10).

3. Dispositif d'administration selon la revendication 1, dans lequel ledit adsorbant à charbon actif est positionné pour retirer un agent stabilisant phénolique de ladite formulation d'insuline avant l'administration au patient.

4. Dispositif d'administration selon la revendication 1, dans lequel ledit adsorbant à charbon actif a un temps de séjour dans ledit élément d'administration pour n'obtenir pratiquement aucune dénaturation ou perte d'efficacité avant l'injection au patient.

5. Dispositif d'administration selon la revendication 3, dans lequel ledit adsorbant à charbon actif comprend un adsorbant à charbon actif traité à l'acide phosphorique.

6. Dispositif d'administration selon la revendication 5, dans lequel ladite cartouche filtrante a une configuration pour fournir un temps de contact de l'insuline avec ledit charbon actif afin de retirer une quantité prédéterminée de phénol et/ou de m-crésol de l'insuline.

7. Dispositif d'administration selon la revendication 1, dans lequel ladite insuline contient un agent stabilisant phénolique, et
ledit dispositif d'administration dirige ladite insuline à travers ledit trajet de fluide, et où ledit adsorbant à charbon actif est inclus en une quantité pour retirer au moins environ 60% en poids de l'agent stabilisant phénolique de ladite formulation d'insuline sur une période d'au moins quatre jours tout en maintenant une activité thérapeutique d'insuline d'au moins environ 73% par rapport à l'insuline non traitée.

8. Dispositif d'administration selon la revendication 7, dans lequel ledit dispositif d'administration est configuré pour une administration continue et contrôlée de ladite insuline pendant une période prédéterminée, et dans lequel ledit adsorbant à charbon actif est inclus en une quantité pour retirer au moins environ 60% en poids de l'agent stabilisant phénolique de ladite formulation d'insuline, et ladite formulation d'insuline administrée à partir dudit ensemble de perfusion présentant une activité thérapeutique d'insuline d'au moins environ 73% après environ 7 jours.

9. Dispositif d'administration selon la revendication 7, dans lequel ledit agent stabilisant phénolique est choisi dans le groupe constitué par le phénol, le m-crésol et des mélanges de ceux-ci.

10. Dispositif d'administration selon la revendication 1 pour administrer de l'insuline, dans lequel ladite insuline comprend un agent stabilisant phénolique en une quantité pour stabiliser ladite insuline.

11. Dispositif d'administration selon la revendication 10, dans lequel ledit charbon actif comprend un charbon actif à l'acide phosphorique.
